# EUROPEAN PATENT SPECIFICATION

(11) **EP 3 003 478 B1**
(45) Date of publication and mention of the grant of the patent: **21.11.2018**
(21) Application number: 14731379.5
(22) Date of filing: 26.05.2014
(51) Int. Cl.: A61N 1/39, G06F 1/32

(54) **AUTOMATIC POWER MANAGEMENT FOR EXTERNAL DEFIBRILLATORS**
AUTOMATISCHE LEISTUNGSVERWALTUNG FÜR EXTERNE DEFIBRILLATOREN
GESTION D'ÉNERGIE AUTOMATIQUE POUR DÉFIBRILLATEURS EXTERNES

(30) Priority: 28.05.2013 US 201361827811 P
(43) Date of publication of application: 13.04.2016
(73) Proprietor: Koninklijke Philips N.V., 5656 AE Eindhoven (NL)
(72) Inventor: DELISLE, Norman Maurice, 5656 AE Eindhoven (NL); WUTHRICH, Scott Alan, 5656 AE Eindhoven (NL); KOZIN, Simon Edward, 5656 AE Eindhoven (NL)
(74) Representative: Steffen, Thomas
(86) International application number: PCT/IB2014/061706
(87) International publication number: WO 2014/191888

(56) References cited:
- WO-A1-02/100483
- US-A1- 2009 182 204
- US-A1- 2012 011 382
- US-B1- 6 586 850

## Description

### BACKGROUND:

### Technical Field

This disclosure relates to medical instruments and more particularly to a defibrillator/monitor with automatic power management to improve battery life, reduce power consumption and reduce startup times for clinical usage.

### Description of the Related Art

An external defibrillator is capable of operating on battery power or on power supplied via an external AC or DC power source. With the current generation of products, the user directly controls power by manually turning the device on and off. Typically, there is a power-on time of at least five to ten seconds as system processors boot up, initialize and perform start up self-test. Additionally, it can take several minutes for some subsystems to warm up to achieve full accuracy.

Newer defibrillator / monitor devices add complications for power control. Modular concepts are being applied to defibrillator / monitor systems so several battery powered modules may be provided that need to be turned on and off by the user. This creates a greater number of steps for the user and opens up the possibility for missing some steps. Valuable time could be lost while the user tries to determine why the system is not operating, e.g., if one or more of the modules is not powered on. In addition, if one of the modules is inadvertently not powered off, it could deplete its battery and not be available for use when needed for emergency care.

US 2012/0011382 discloses a system and method for conservation of battery power in
a portable medical device, which uses a general purpose processor with a sleep state. The sleep state is used when a critical purpose processor does not detect a critical event.

### SUMMARY

The invention is defined by the claims. In accordance with the present principles, an emergency medical device includes a power source configured to power one or more subsystems of the device. A user activity detection module is configured to sense user activity in the one or more subsystems, and a clinical activity detection module is configured to sense clinical activity in the one or more subsystems. A control module is coupled to the one or more subsystems and configured to alter a power status of the one or more subsystems in accordance with an activity sensed in at least one of the user activity detection module and the clinical activity detection module. An emergency medical device includes a power source configured to power one or more subsystems of the device, the one or more subsystems including at least one module having a standby mode; and a control module coupled to the one or more subsystems and including an automatic power management module. The control module is configured to control parameters for changing a power status of the one or more subsystems. The power status of the one or more subsystems is altered in accordance with an activity sensed in at least one of a user activity detection module and a clinical activity detection module wherein the user activity detection module is configured to sense user activity in the one or more subsystems, and the clinical activity detection module is configured to sense clinical activity in the one or more subsystems.

A method for power management of a medical device includes configuring a control module to be responsive to one or more user activities and/or one or more clinical activities performed on the device; sensing user activities and clinical activities to determine whether conditions are met for altering a power status of at least one of the defibrillator and one or more subsystems of the defibrillator; and altering the power status of the at least one of the
device overall and the one or more subsystems in accordance with the user activities and clinical activities sensed.

The power status can include one of powered down, powered up and standby mode. The one or more subsystems can include a measurement module, a printer, and a display. User activities can include physical interactions between a user and the device and the clinical activities include clinical interactions with a patient, preparing equipment for the patient and measuring patient parameters. Configuring the control module can include permitting a user to set parameters for altering the power status. The device can include a monitor, a defibrillator or a combination thereof.

These and other objects, features and advantages of the present disclosure will become apparent from the following detailed description of illustrative embodiments thereof, which is to be read in connection with the accompanying drawings.

### BRIEF DESCRIPTION OF DRAWINGS

This disclosure will present in detail the following description of preferred embodiments with reference to the following figures wherein:
FIG. 1 is a block/flow diagram showing a defibrillator/monitor device in accordance with one embodiment;
FIG. 2 is a block/flow diagram showing a power control module and modules powered thereby for a defibrillator in accordance with one embodiment; and
FIG. 3 is a block/flow diagram showing a system/method for operating a defibrillator in accordance with an illustrative embodiment.

### DETAILED DESCRIPTION OF EMBODIMENTS

In accordance with the present principles, an automated power management capability is provided to mitigate usability issues with powering on a defibrillator or monitor making an overall system easier to use and improving system readiness for emergency care. Current technologies for automated power management are not a good fit for the usage patterns of a defibrillator or monitor. User inactivity is a primary trigger that many consumer electronic devices use to enter a power-saving mode. The power saving modes will remove power from a display screen, one of the largest consumers of power in the device. In a defibrillator or monitor, vital signs data displayed on the screen might be needed even during long periods of apparent user inactivity. In addition, users rely on the defibrillator / monitor to continuously check for physiological alarm conditions such as a high heart rate, a low blood pressure or a more complex condition such as an arrhythmia detected in the patient's ECG. Therefore, simple user inactivity metrics cannot be relied on for automatic power management of a defibrillator/monitor.

In accordance with useful embodiments, clinical activity is employed in addition to user activity to trigger an automatic power saving mode switching. Clinical activity measures may include one or more of the following: 1) therapy pads or paddles being attached from the device to the patient, 2) electrocardiogram (ECG) leads being attached from the device to the patient, valid ECG data being received from pads, paddles or ECG leads, a valid Pleth wave signal being received from an SpO₂ sensor, a valid capnogram being received from an EtCO₂ sensor, compressions detected on a cardiopulmonary resuscitation (CPR) coaching sensor connected to the device, activating a measurement system, etc.

A level of clinical activity used to trigger powering off of certain subsystems needs to be different than those for powering on those subsystems. The rules need to provide a high threshold for powering off to prevent false inactivity detections. The rules for reawakening a subsystem use a lower threshold to ensure that all activity conditions are detected. As an example, attaching ECG leads to the device is a sufficient condition for powering on ECG monitoring functions. However, to power off the ECG monitoring functions may call for detaching both ECG leads and other ECG sources such as pads or paddles. User activity includes any direct or affirmative control of the device such as turning a knob, pressing a button or undocking a module for remote usage, etc.

The device enters a power-saving mode only if there is no user activity and no clinical activity for a predefined time period. While in the power saving mode of operation, the device continues to sense user activity and certain types of clinical activity to determine whether the device needs to be automatically reawakened.

Mechanisms for detecting user or clinical activity are designed to have very low power consumption. Note that with a standby mode of operation, where the device can be instantly awakened, the processors and memory need to continue to be powered. Therefore, user and clinical activity detection mechanisms can utilize these active processing components. For example, the device can detect when the pads cable becomes connected to the device by using a simple input/output method to detect when a load is connected between specific therapy port pins.

A system in accordance with the present principles provides instant-on capability while operating in the power saving modes. Therefore, recovery is instantaneous if the system enters its power saving mode just prior to a new occurrence of user activity or clinical activity. Automated power management provides a high level of robustness that is needed in an emergency medical device because automated power saving actions taken by the device can be instantly reversed by the user via the instant-on feature. In one embodiment, multiple power saving modes and switches based on specific types of inactivity may be employed. For example, the power to a printer is turned off if there have been no printing requests (from the user or from auto-print capabilities such as an arrhythmia alarm). Power to the printer is restored if a print request occurs, which is all transparent to the user. For long-term patient monitoring as in patient transport, significant battery power is saved with a power saving mode to turn off the display and printer, but continue to monitor for patient alarm conditions and automatically reawaken the system if an alarm condition is detected.

It should be understood that the present invention will be described in terms of medical instruments; however, the teachings of the present invention are much broader and are applicable to any battery operated device that needs to reduce power consumption. In some embodiments, the present principles are employed in defibrillators/monitors used in hospitals or emergency vehicles, in homes, in public places, etc. The elements depicted in the FIGS. may be implemented in various combinations of hardware and software and provide functions which may be combined in a single element or multiple elements.

The functions of the various elements shown in the FIGS. can be provided through the use of dedicated hardware as well as hardware capable of executing software in association with appropriate software. When provided by a processor or controller, the functions can be provided by a single dedicated processor, by a single shared processor, or by a plurality of individual processors, some of which can be shared. Moreover, explicit use of the term "processor" or "controller" should not be construed to refer exclusively to hardware capable of executing software, and can implicitly include, without limitation, digital signal processor ("DSP") hardware, read-only memory ("ROM") for storing software, random access memory ("RAM"), non-volatile storage, etc.

Moreover, all statements herein reciting principles, aspects, and embodiments of the invention, as well as specific examples thereof, are intended to encompass both structural and functional equivalents thereof. Additionally, it is intended that such equivalents include both currently known equivalents as well as equivalents developed in the future (i.e., any elements developed that perform the same function, regardless of structure). Thus, for example, it will be appreciated by those skilled in the art that the block diagrams presented herein represent conceptual views of illustrative system components and/or circuitry embodying the principles of the invention. Similarly, it will be appreciated that any flow charts, flow diagrams and the like represent various processes which may be substantially represented in computer readable storage media and so executed by a computer or processor, whether or not such computer or processor is explicitly shown.

Furthermore, embodiments of the present invention can take the form of or include a computer program product accessible from a computer-usable or computer-readable storage medium providing program code for use by or in connection with a computer or any instruction execution system. For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system (or apparatus or device) or a propagation medium. Examples of a computer-readable medium include a semiconductor or solid state memory, magnetic tape, a removable computer diskette, a random access memory (RAM), a read-only memory (ROM), a rigid magnetic disk and an optical disk. Current examples of optical disks include compact disk - read only memory (CD-ROM), compact disk - read/write (CD-R/W), Blu-Ray™ and DVD.

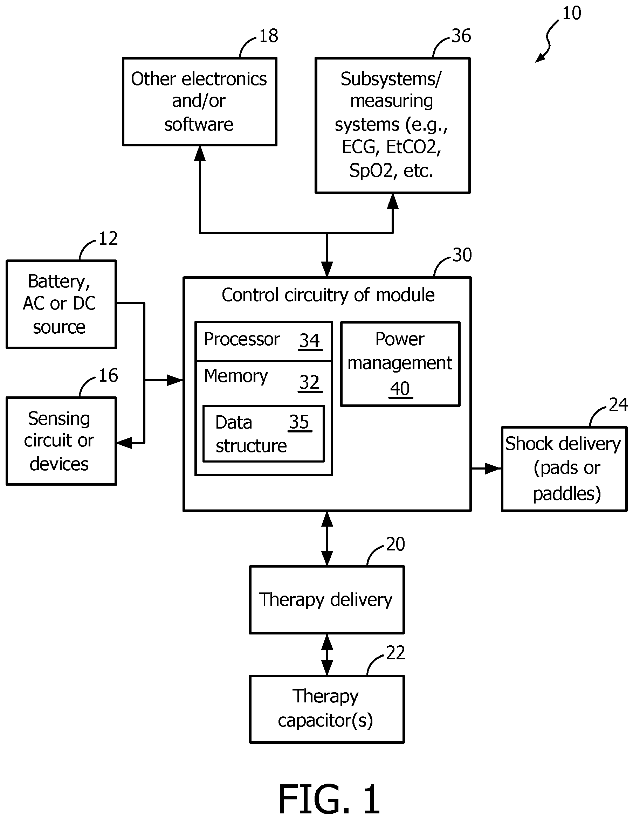

Referring now to the drawings in which like numerals represent the same or similar elements and initially to FIG. 1, a block/flow diagram shows an emergency medical device 10 such as a defibrillator, monitor or combination thereof in accordance with one embodiment. Defibrillator/monitor device 10 includes a power source 12, such as, e.g., a battery or batteries, a DC power source or an AC power source. Therapy delivery circuitry 20 controls the amount of energy and shapes of pulses delivered to shock delivery devices 24, which may include pads or paddles. Therapy delivery circuitry 20 provides access to therapy capacitors 22 to enable charging and discharging thereof. Defibrillator/monitor device 10 may include other electronics or software programs 18. The electronics 18 may include other features, such as clocks, timers, power measurement devices, a light, a display, etc. Monitoring device functions 36 may include electrocardiography (ECG) devices, oxygen saturation devices (SpO2), non-invasive blood pressure devices (NBP), capnography devices (EtCO2), temperature devices and others.

When the defibrillator/monitor device 10 needs to deliver a shock, the therapy delivery circuitry 20 charges the therapy capacitor(s) 22 by routing current from the battery or batteries 12 (or an AC or DC power source). When a user triggers the release of energy, the energy stored in therapy capacitor(s) 22 is discharged via pads or paddles 24 placed on a patient's chest.

Defibrillator/monitor device 10 includes a control circuit or module 30, which may include one or more processors 34 and memory 32. The control module 30 controls operations and functions of the other modules or features of the defibrillator/monitor 10.

Defibrillator/monitor device 10 includes a combination of automatic power management mechanisms 40 that simplify the usage of defibrillator / monitor systems and save energy to extend battery operating times. Defibrillator/monitor device 10 provides power-saving standby modes for selected subsystems with instant power on capabilities to avoid lengthy processor boot-up time where applicable (e.g., booting a real-time operating system on a system processor or computer processing unit (CPU) 34). The defibrillator/monitor device 10 avoids or reduces lengthy warm up times where applicable (e.g., end tidal CO₂ measurement module) and provides low-power mechanisms (40) to monitor for user or clinical activity using a sensing circuit or devices 16. The defibrillator/monitor device 10 provides independent power control for selected modules that can be powered on without significant boot up or warm up time (e.g., a printer, non-invasive blood pressure measurement module, etc.).

Low power mechanisms 40 for detecting user or clinical activities may include user activities such as pressing a button, touching a touch-screen, turning a knob, connecting a therapy or measurement cable to the device, undocking of separable system modules, etc. Clinical activities may include placing ECG leads on a patient, placing therapy pads or paddles 24 on the patient, acquiring a valid ECG signal, or detecting a chest compression via a cardiopulmonary resuscitation (CPR) sensor, etc.

The defibrillator/monitor device 10 is configured to automatically switch out of standby mode (instant on) and power on modules when a user activity and a clinical activity is detected. The defibrillator/monitor device 10 is also configured to automatically switch into standby mode and power off modules after an interval of both user inactivity and clinical inactivity. In other embodiments, powering off or switching to standby power is selectively performed for portions of the system for certain restricted types of user inactivity and clinical inactivity. For example, a printer is powered off when there are no print requests, manually controlled vital signs measurement modules are powered off when there is no activity (e.g., non-invasive blood pressure module), vital signs measurement modules are switched to power-saving standby mode when they are not connected to the patient (e.g., no sensor cable connected to end-tidal CO₂ module), therapy delivery circuitry is powered off when the device is operating in a mode where therapy cannot be delivered (e.g., shocks cannot be delivered in monitor mode).

The combination of these automatic power management features 40 ensures the device can be activated instantly when needed, the device does not accidently power off when in use, and battery power consumption is reduced.

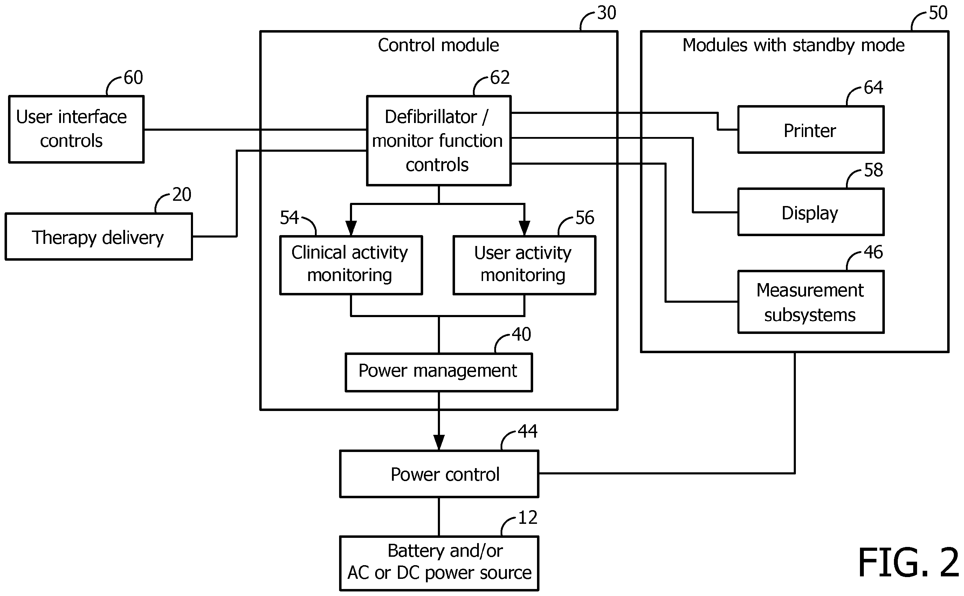

Referring to FIG. 2, a block diagram shows an exemplary hardware embodiment for control module 30 and power management module/automatic power management module 40 in accordance with the present principles. The control module 30 provides processing, memory and software that controls device functionality and controls operating state caused transitions from "on" to "standby", among other things. A power control 44 includes hardware for turning on/off specific subsystems, e.g., switches and connectors.

The control module 30 includes the automatic power management module 40. The automatic power management module 40 receives input from a plurality of different modules to determine whether the power control module 44 should be activated to power up any number of independently powered device subsystems 46 or other devices, e.g., printer 64, therapy delivery 20, display 58, etc.

An illustrative example of this includes detecting user activity such as pushing a front panel button. User interface control 60 detects the button press and signals the control module 30. This signal is processed by a defibrillator/monitor function control module 62. In addition, this signal is passed to a user activity monitoring module 56 to determine whether this button press activates the device (if the device was in standby state), one or more subsystems or resets inactivity timers.

An illustrative example of clinical activity monitoring includes measurement subsystems 46 that acquire and process vital signs measurement data and send the data to the defibrillator/monitor function control 62 for display, etc. In addition, clinical actions such as initial acquisition of a valid ECG signal are signaled to a clinical activity monitoring module 54 to determine whether the acquisition activates the device (if the device was in standby state), one or more subsystems or resets inactivity timers.

Modules 50 with a standby mode and/or modules with independent power control are controlled using the function control module 62. The power control module 44 controls standby modes for modules 50 and switches power on or off independently for each subsystem 46, display 58, printer 64, etc. The automatic power management module 40 provides timers for user activities and clinical activity, and receives input from user activity detection devices or circuits 56 and from clinical activity monitoring devices and circuits 54. When inactivity period thresholds are exceeded, subsystems 46 are switched to standby mode or powered off according to the capabilities of the subsystems 46. Likewise, when a new user or clinical activity is detected in blocks 56 and/or 54, corresponding subsystems 46 or other devices are automatically turned on.

Subsystems 46 of the defibrillator/monitor device 10 may include, e.g., an end tidal CO₂ measurement module, a printer 64, a non-invasive blood pressure measurement module, a display 58, vital signs measurement modules, therapy delivery circuitry, etc. Each subsystem 46 may include local sensors to measure user or clinical activity specific to that subsystem 46. When the subsystem 46 is determined to be inactive, the subsystem 46 is either powered off or enters standby mode depending of the capabilities and advantages of doing so for that subsystem 46. Powering down or standby mode may be reversed upon an additional user or clinical activity or combination of both. For example, a user activity (e.g., plugging in the leads for the paddles) and a clinical activity (e.g., placing therapy pads or paddles 24 on the patient) may both be needed to switch out of standby mode.

In one embodiment, the combinations and the manner of power down and standby modes (e.g., inactivity times, etc.) can be programmed by a user using the user interface 60. The interface 60 may include the display 58 (which may include a touch screen) and/or another data entry device 60, such as a buttons, knobs, keyboard, mouse, joystick, etc. Note that the display 58, processor 34, etc. may include power down modes as well and be controlled in accordance with the automatic power management module 40.

A data structure 35 may be stored in memory 32 (FIG. 1) to enable the set up of different conditions and alarms specific for each subsystem. These conditions and alarms may be customized by the user or default parameters may be employed. The parameters may include a type of activity, duration of use or inactivity, a frequency of use or any other pertinent information useful in determining an activity state of the device 10 as a whole or any one or more of the subsystems of the device 10. Others parameters are contemplated as well.

In addition to controlling the power to individual subsystems 46, an overall operational state of the device 10 can be controlled as a unit. For example, all subsystems that have standby capability can be powered on or off at the same time based on any system-level activity or inactivity.

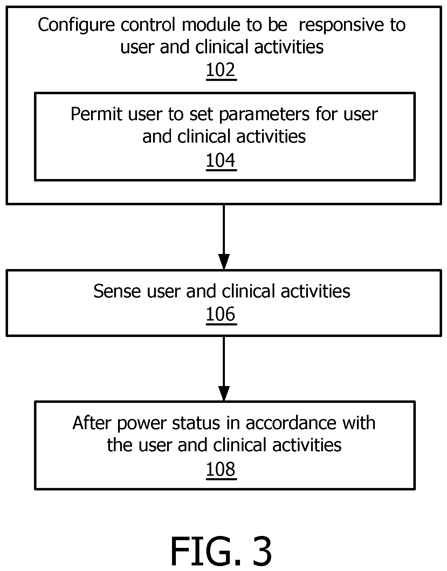

Referring to FIG. 3, a block/flow diagram shows a method for power management of a defibrillator/monitor in accordance with the present principles. In block 102, a control module is configured to be responsive to one or more user activities and/or one or more clinical activities performed on the defibrillator. This may include set up a data structure (35), e.g., a lookup table or the like, in memory (32) to index the activities detected against actions to take. In this way, a number of power down, power up and/or standby modes may be set for the defibrillator as a whole or for the one or more subsystems. In block 104, a user is permitted to set parameters for altering the power status or to modify default settings.

In block 106, user activities and clinical activities are sensed to determine whether conditions are met for altering a power status of at least one of the defibrillator and one or more subsystems of the defibrillator. The one or more subsystems may include a measurement module, a printer, a display, etc. Other subsystems are also contemplated. The power status may include one of powered down, powered up and standby mode. The status may also include a measure of how much power has been reduced, e.g., in the form of a percentage.

In block 108, the power status of the at least one of the defibrillator and the one or more subsystems may be altered or modified in accordance with the user activities and clinical activities sensed. The user activities include physical interactions between a user and the defibrillator, and the clinical activities include interactions with the patient, preparing equipment for the patient and measuring patient parameters. Other activities are also contemplated.

In interpreting the appended claims, it should be understood that:
a) the word "comprising" does not exclude the presence of other elements or acts than those listed in a given claim;
b) the word "a" or "an" preceding an element does not exclude the presence of a plurality of such elements;
c) any reference signs in the claims do not limit their scope;
d) several "means" may be represented by the same item or hardware or software implemented structure or function; and
e) no specific sequence of acts is intended to be required unless specifically indicated.

Having described preferred embodiments for automatic power management for external defibrillators (which are intended to be illustrative and not limiting), it is noted that modifications and variations can be made by persons skilled in the art in light of the above teachings. It is therefore to be understood that changes may be made in the particular embodiments of the disclosure disclosed which are within the scope of the embodiments disclosed herein as outlined by the appended claims. Having thus described the details and particularity required by the patent laws, what is claimed and desired protected by Letters Patent is set forth in the appended claims.

## Claims

1. An emergency medical device with automatic power management, comprising:
one or more subsystems including at least one module having a standby mode;
a power source (12) configured to power one or more subsystems of the device;
a user activity detection module (56) configured to sense user activity in the one or more subsystems, the user activity consisting of turning a knob, touching a touch screen, connecting a therapy or measurement cable to the device, pressing a button or undocking a module for remote usage;
a clinical activity detection module (54) configured to sense clinical activity in the one or more subsystems, the clinical activity consisting of:
therapy pads or paddles being attached from the device to the patient,
electrocardiogram leads being attached from the device to the patient,
valid ECG data being received from pads, paddles or ECG leads,
a valid Pleth wave signal being received from an SpO₂ sensor,
a valid capnogram being received from an EtCO₂ sensor or
compressions detected on a cardiopulmonary resuscitation (CPR) coaching sensor connected to the device; **characterised in** further comprising:
a control module (30) coupled to the one or more subsystems and configured to automatically switch out of standby mode and power on the module of the one or more subsystems in response to a clinical activity in addition to a user activity being detected.

2. The device as recited in claim 1, wherein the control module (30) includes an automatic power management module (40) configured to control parameters for changing a power status of the one or more subsystems.

3. The device as recited in claim 2, further comprising a power control module (44) configured to modify the power status of the one or more subsystems based upon input from the automatic power management module.

4. The device as recited in claim 3, wherein the automatic power management module (40) is configured to control independently powering down the one or more subsystems in accordance with inactivity sensed in at least one of the user activity detection module and the clinical activity detection module.

5. The device as recited in claim 1, wherein the one or more subsystems (46) includes one or more of a measurement module, a printer, and a display.

6. The device as recited in claim 1, further comprising an interface (60) configured to permit a user to set parameters for altering the power status of the one or more subsystems.

7. The device as recited in claim 1, wherein an overall operational state of the device is controllable as a unit such that all subsystems that have standby capability are powered on or off at a same time based on any system-level activity or inactivity.

8. The device as recited in claim 1, wherein the device includes a monitor, a defibrillator or a combination thereof.

9. A method for power management of a medical device, comprising one or more subsystems including at least one module having a standby mode, the method compring:
configuring (102) a control module to be responsive to one or more user activities and one or more clinical activities performed on the device, the user activities consisting of turning a knob, touching a touch screen, connecting a therapy or measurement cable to the device, pressing a button or undocking a module for remote usage, and the clinical activities consisting of therapy pads or paddles being attached from the device to the patient, electrocardiogram leads being attached from the device to the patient, valid ECG data being received from pads, paddles or ECG leads, a valid Pleth wave signal being received from an SpO₂ sensor, a valid capnogram being received from an EtCO₂ sensor or compressions detected on a cardiopulmonary resuscitation (CPR) coaching sensor connected to the device;
sensing (106) user activities and clinical activities to determine whether conditions are met for automatically switching out of standby mode and power on the module of the one or more subsystems of the device; and
automatically switching out of standby mode and power on the module of the one or more subsystems in response to the user activities and clinical activities being sensed.

## Patentansprüche

1. Medizinisches Notfallgerät mit automatischer Energieverwaltung, umfassend:
eines oder mehrere Teilsysteme umfassend mindestens ein Modul mit Standby-Modus;
eine Stromquelle (12), die konfiguriert ist, um eines oder mehrere Teilsysteme des Geräts mit Strom zu versorgen;
ein Benutzeraktivitätserfassungsmodul (56), das konfiguriert ist, um Benutzeraktivitäten in einem oder mehreren Teilsystemen zu erfassen, wobei die Benutzeraktivität darin besteht, einen Drehknopf zu drehen, einen Touchscreen zu berühren, ein Therapie- oder Messkabel mit dem Gerät zu verbinden, eine Taste zu drücken oder ein Modul für die Fernsteuerung abzukoppeln;
ein Modul zur Erfassung der klinischen Aktivitäten (54), das konfiguriert ist, um die klinischen Aktivitäten in einem oder mehreren Teilsystemen zu erfassen, wobei die klinischen Aktivitäten bestehen aus:
Befestigen von Therapie-Pads oder -Paddeln vom Gerät am Patienten,
Befestigen von Elektrokardiogrammleitungen vom Gerät am Patienten,
Empfang valider EKG-Daten von Pads, Paddeln oder EKG-Leitungen,
Empfang eines validen Pleth-Wellensignals von einem SpO₂ -Sensor,
Empfang eines validen Kapnogramms von einem EtCO₂ Sensor oder
von Kompressionen, die an einem an das Gerät angeschlossenen Coachingsensor für Herz-Lungen-Wiederbelebung (CPR) detektiert werden; **dadurch gekennzeichnet, dass** es weiter umfasst:
ein Steuermodul (30), das mit dem einen oder den mehreren Teilsystemen gekoppelt ist und so konfiguriert ist, dass es automatisch aus dem Standby-Modus wechselt und das Modul des einen oder der mehreren Teilsysteme als Reaktion auf eine klinische Aktivität, zusätzlich zur Erfassung einer Benutzeraktivität, einschaltet.

2. Gerät nach Anspruch 1, wobei das Steuermodul (30) ein automatisches Energieverwaltungsmodul (40) umfasst, das konfiguriert ist, um Parameter zum Ändern eines Energiestatus des einen oder der mehreren Teilsysteme zu steuern.

3. Gerät nach Anspruch 2, weiter umfassend ein Energiesteuerungsmodul (44), das konfiguriert ist, um den Energiestatus des einen oder der mehreren Teilsysteme auf der Grundlage der Eingaben des automatischen Energiesteuerungsmoduls zu ändern.

4. Gerät nach Anspruch 3, wobei das automatische Energieverwaltungsmodul (40) so konfiguriert ist, dass es das Herunterfahren des einen oder der mehreren Teilsysteme gemäß der in mindestens einem der Benutzeraktivitätserkennungsmodule und dem klinischen Aktivitätserkennungsmodul erfassten Inaktivität steuert.

5. Gerät nach Anspruch 1, wobei das eine oder die mehreren Teilsysteme (46) ein oder mehrere von einem Messmodul, einem Drucker und einer Anzeige umfassen.

6. Gerät nach Anspruch 1, weiter umfassend eine Schnittstelle (60), die so konfiguriert ist, dass ein Benutzer Parameter zum Ändern des Energiestatus des einen oder der mehreren Teilsysteme einstellen kann.

7. Gerät nach Anspruch 1, wobei ein Gesamtbetriebszustand des Gerätes als Einheit derart steuerbar ist, dass alle Teilsysteme, die Standby-fähig sind, aufgrund einer Aktivität oder Inaktivität auf Systemebene gleichzeitig ein- oder ausgeschaltet werden.

8. Gerät nach Anspruch 1, wobei das Gerät einen Monitor, einen Defibrillator oder eine Kombination davon umfasst.

9. Verfahren zur Energieverwaltung eines medizinischen Gerätes, umfassend ein oder mehrere Teilsysteme mit mindestens einem Modul mit einem Standby-Modus, wobei das Verfahren umfasst:
Konfigurieren (102) eines Steuermoduls, sodass es auf eine oder mehrere Benutzeraktivitäten und eine oder mehrere am Gerät durchgeführten klinischen Aktivitäten reagiert, wobei die Benutzeraktivitäten darin bestehen, einen Knopf zu drehen, einen Touchscreen zu berühren, ein Therapie- oder Messkabel an das Gerät anzuschließen, eine Taste zu drücken oder ein Modul für die Fernsteuerung abzukoppeln, und wobei die klinischen Aktivitäten daraus bestehen, Therapie-Pads oder -Paddel vom Gerät am Patienten zu befestigen, Elektrokardiogrammleitungen vom Gerät am Patienten zu befestigen, valide EKG-Daten von Pads, Paddeln oder EKG-Leitungen zu empfangen, ein valides Pleth-Wellensignal von einem SpO₂ -Sensor zu empfangen, ein valides Kapnogramm von einem EtCO₂ -Sensor zu empfangen, oder Kompressionen an einem an das Gerät angeschlossenen Coachingsensor für Herz-Lungen-Wiederbelebung (CPR) zu erfassen;
Erfassen (106) von Benutzeraktivitäten und klinischen Aktivitäten, um festzustellen, ob die Bedingungen für das automatische Wechseln aus dem Standby-Modus das Einschalten des Moduls des einen oder der mehreren Teilsysteme des Geräts erfüllt sind; und
automatisches Wechseln aus dem Standby-Modus und Einschalten des Moduls eines oder mehrerer Teilsysteme als Reaktion auf die erfassten Benutzeraktivitäten und klinischen Aktivitäten.

## Revendications

1. Dispositif d'urgence médical avec une gestion d'énergie automatique, comprenant :
un ou plusieurs sous-systèmes incluant au moins un module ayant un mode veille ;
une source d'énergie (12) configurée pour alimenter un ou plusieurs sous-systèmes du dispositif ;
un module de détection de l'activité d'un utilisateur (56) configurée pour capter l'activité d'un utilisateur dans le ou les sous-systèmes, l'activité d'un utilisateur étant constituée du réglage d'un bouton, du touché d'un écran tactile, de la connexion d'un câble de thérapie ou de mesure au dispositif, de la pression d'un bouton ou du déverrouillage d'un module pour une utilisation éloignée ;
un module de détection d'une activité clinique (54) configurée pour capter une activité clinique dans le ou les sous-systèmes, l'activité clinique étant constituée :
d'électrodes ou de palettes de thérapie fixées du dispositif sur le patient,
de fils d'électrocardiogramme fixés du dispositif sur le patient,
de données d'ECG valides reçues des électrodes, des palettes ou des fils d'ECG,
d'un signal d'onde de Pleth valide reçu d'un capteur de PsO₂,
d'un capnogramme valide reçu d'un capteur d'EtCO₂ ou
de compressions détectées sur un capteur d'entraînement de réanimation cardio-respiratoire (RCR) connecté au dispositif ; **caractérisé en ce qu'**il comprend en outre :
un module de commande (30) couplé au ou aux sous-systèmes et configuré pour éteindre automatiquement le mode veille et allumer le module du ou des sous-systèmes en réponse à une activité clinique en plus d'une activité d'un utilisateur détectée.

2. Dispositif selon la revendication 1, dans lequel le module de commande (30) inclut un module de gestion d'énergie automatique (40) configuré pour commander les paramètres pour un changement d'état d'énergie du ou des sous-systèmes.

3. Dispositif selon la revendication 2, comprenant en outre un module de commande d'énergie (44) configuré pour modifier l'état d'énergie du ou des sous-systèmes sur la base d'une entrée provenant du module de gestion d'énergie automatique.

4. Dispositif selon la revendication 3, dans lequel le module de gestion d'énergie automatique (40) est configuré pour commander indépendamment la mise hors tension du ou des sous-systèmes en fonction de l'inactivité captée par l'un au moins parmi le module de détection de l'activité d'un utilisateur et le module de détection d'une activité clinique.

5. Dispositif selon la revendication 1, dans lequel le ou les sous-systèmes (46) incluent un ou plusieurs parmi un module de mesure, une imprimante et un écran.

6. Dispositif selon la revendication 1, comprenant en outre une interface (60) configurée pour permettre à un utilisateur de fixer des paramètres pour modifier l'état d'énergie du ou des sous-systèmes.

7. Dispositif selon la revendication 1, dans lequel l'état opérationnel global du dispositif est contrôlable en tant qu'unité de sorte que tous les sous-systèmes qui ont une capacité de veille soient allumés ou éteints en même temps sur la base de toute activité ou inactivité du niveau du système.

8. Dispositif selon la revendication 1, dans lequel le dispositif inclut un moniteur, un défibrillateur ou une combinaison de ceux-ci.

9. Procédé de gestion d'énergie d'un dispositif médical, comprenant un ou plusieurs sous-systèmes incluant au moins un module ayant un mode veille, le procédé comprenant le fait de :
configurer (102) un module de commande devant répondre à une ou plusieurs activités d'un utilisateur et à une ou plusieurs activités cliniques réalisées sur le dispositif, les activités d'un utilisateur étant constituées du réglage d'un bouton, du touché d'un écran tactile, de la connexion d'un câble de thérapie ou de mesure au dispositif, de la pression d'un bouton ou du déverrouillage d'un module pour une utilisation éloignée ; et les activités cliniques étant constituées d'électrodes ou de palettes de thérapie fixées du dispositif sur le patient, de fils d'électrocardiogramme fixés du dispositif sur le patient, de données d'ECG valides reçues des électrodes ou des palettes ou des fils d'ECG, d'un signal d'onde de Pleth valide reçu d'un capteur de PsO₂, d'un capnogramme valide reçu d'un capteur d'EtCO₂ ou de compressions détectées sur un capteur d'entraînement de réanimation cardio-respiratoire (RCR) connecté au dispositif ;
capter (106) des activités d'un utilisateur et des activités cliniques pour déterminer si les conditions sont réunies pour éteindre automatiquement le mode veille et allumer le module du ou des sous-systèmes du dispositif ; et
éteindre automatiquement le mode veille et allumer le module du ou des sous-systèmes en réponse aux activités d'un utilisateur et aux activités cliniques détectées.
